# EUROPEAN PATENT SPECIFICATION

(11) **EP 2 675 728 B1**
(45) Date of publication and mention of the grant of the patent: **30.12.2020**
(21) Application number: 12747541.6
(22) Date of filing: 14.02.2012
(51) Int. Cl.: B65D 83/04, A61J 1/03, A61J 7/04, H05K 1/16, G09F 3/03, G08C 17/00

(54) **MONITORING SYSTEM FOR MONITORING SMART PACKAGE CONTENT USE**
ÜBERWACHUNGSSYSTEM ZUR ÜBERWACHUNG DER INTELLIGENTEN VERWENDUNG VON PAKETINHALTEN
SYSTÈME DE SURVEILLANCE PERMETTANT DE SURVEILLER L'UTILISATION DU CONTENU D'UN EMBALLAGE INTELLIGENT

(30) Priority: 15.02.2011 FI 20115146
(43) Date of publication of application: 25.12.2013
(73) Proprietor: Stora Enso Oyj, 00101 Helsinki (FI)
(72) Inventor: MAIJALA, Juha, FI-04500 Kellokoski (FI); MÄKELÄ, Raimo, FI-08500 Lohja (FI); ILKKA, Petri, FI-37100 Nokia (FI)
(74) Representative: Berggren Oy, Helsinki & Oulu
(86) International application number: PCT/FI2012/050141
(87) International publication number: WO 2012/110700

(56) References cited:
- EP-A1- 2 243 461
- WO-A1-02/05039
- WO-A1-2010/066456
- WO-A2-2007/043858
- US-A1- 2004 178 112
- US-A1- 2004 178 112
- US-A1- 2007 204 691
- US-A1- 2009 277 815
- US-A1- 2010 089 791

## Description

The invention relates to a monitoring system for monitoring the use of smart package content, as well as to a method for monitoring such use of smart package content.

### PRIOR ART

The prior art discloses various arrangements for monitoring the content use of medication packages, such as for example a solution presented by Wachnam et al. in his publication US 2010/0270257, wherein the cap of a medication bottle is provided with detection elements for monitoring opening of the cap. As a result of opening, the cap of a medication bottle is in data communication, for example by way of a modem, with a third party, such as a database.

A problem with such solutions is that medication packages and detection elements presented therein are highly complicated and expensive to use. In addition, they are not very friendly in terms of environment, because, among others, the above-mentioned cap of a medication bottle contains a lot of high standard electronics with its inherent display units and components. As a result, such packages are not recyclable or for example cannot be incinerated among normal combustible waste, because the electronic components used therein contain metals and other environmentally harmful substances. In addition, such packages are inconvenient to carry along, being bulky and requiring for example the use of a modem. Still furthermore, they are quite easily cheated, because the monitoring system is not for example capable of distinguishing whether a medicine was extracted from the medication bottle or whether the cap was merely opened for no particular purpose. In addition, the cap can be opened by someone other than the intended user of the medication container, nor can the system be totally certain as to who actually opened the cap.

Publication US 2004/0178112 A1 presents a child resistant medication package and associated System can enable compliance and anti-diversion features. The medication package can include a timing reminder, can detect and record a compliance history of each dose of medication, and can include anti-tamper capabilities to ensure doses are removed in the intended fashion. The medication package can have the capability to exchange data with other devices, including personal computers. The associated system can include a central database that receives data from the medication package, and provides for the transmission of data between various system locations, including not only the individual medication package, but also, for example, drug manufacturers, pharmacies, physician offices, law enforcement agencies, and a database managing agency.

Publication WO 2010/066456 A1 presents a dispenser for dispensing in a unit dose manner. The dispenser comprises components of a reservoir having compartments, a positioning disc having exit orifices. and a guiding member being rotatably arranged in between the reservoir and the positioning disc and respectively connecting one compartment with one dedicated exit orifice in dispensing communication by accomplishing a rotational step.

### SUMMARY

It is one objective of the invention to eliminate or at least alleviate drawbacks related to the prior art. The invention pursues to provide a monitoring system as environmentally friendly as possible, which would nevertheless be as simple and convenient as possible to implement, and that a smart medication package used in the system would be recyclable after use. Another further objective is to provide such a monitoring system, which could make certain at a higher probability that it was definitely the intended user of the medication package who took a medicine, and that the medicine was definitely extracted out of the package. Another objective is that monitoring would be highly transparent for the user and that the system would be easy and inconspicuous to use as one of the normal daily chores.

The invention is defined by the appended claims.

The monitoring system of the invention is characterized by what is presented in claim 1 directed to a monitoring system. The method of the invention for monitoring smart package content use is characterized by what is presented in claim 8 directed to a method.

The invention relates to a monitoring system for monitoring smart package content use, wherein the monitoring system comprises:
- a smart package of recyclable material, configured to comprise removable content units as well comprising a data transfer module functional in a mobile communication network and a docking unit of recyclable material for docking said data transfer module to the package in releasable and electrical manner for the duration of package use, and
- database elements.

The smart package is adapted to detect said removal of a content unit and to supply information to said database elements by way of said communication elements regarding the removal of said content unit. Said smart package is identifiable by the database elements on the basis of information delivered thereby over said communication elements. The smart package may be capable of transmitting for example a package identifying character string, or optionally its communication elements may transmit a distinct identification message in conjunction with the transmission of information.

The database elements comprise means for linking the smart package identification information to the smart package user in the database elements, as well as elements for linking a smart package content use instruction to said smart package identification information in the database elements, as well as communication elements for delivering content use relating instructions either to the smart package and/or to the user's other terminal unit, in which case the monitoring system comprises also said terminal unit.

The monitoring system is adapted to compare location data of both the smart package and the user's other terminal unit and to produce an alarm in the event that said pieces of location data deviate from each other more than what has been prescribed, at a time when, according to the smart package content use instruction, some contents of the smart package should have been removed from said smart package.

According to one embodiment of the invention, the monitoring system has its smart package provided with a specific sensing element for every content unit, such as a pill, for detecting a removal of this particular pill. The smart package is most preferably adapted to detect and identify, by means of said sensing elements, the removal of a specific pill corresponding to a sensing element, and to deliver information, by means of the communication elements, relating to the removal of just this particular pill. The sensing elements are most preferably provided on a backing of the smart package, upon which they can be pressed by methods of printing technology.

According to one embodiment of the invention, the pill-designated sensing element comprises a component, such as a resistor, capacitor, switch, and/or conductor line, which is most preferably fabricated by methods of printing technology. The specific electrical values, such as resistance or capacitance, of at least two printed components present in the backing can be arranged, for example with methods of printing technology, to be different from each other, such that the removal of a pill brings about a change in the structure of a particular component, whereby the removable content unit producing said change can be identified on the basis of the magnitude of a change in the specific electrical value inherent to said component, such as for example a change in resistance or capacitance.

In addition, according to one embodiment of the invention, the communication elements comprise most preferably wireless communication elements, such as a GSM, GPRS or UMTS module, or some other data transfer module functional in a data transfer network, comprising also more recent data transfer technologies, such as 3G and 4G techniques. The smart package may further comprise a smart package identifying element, such as ID information stored in a memory, or for example a SIM card connectable to a data transfer module.

The smart package may additionally comprise also positioning elements, such as for example a GPS receiver, or most preferably a module receiving location data delivered by the cellular information of a mobile communication network. Said module can be for example the above-mentioned data transfer module operating in a mobile communication network, for example a GSM module or the like.

According to one embodiment of the invention, the monitoring system's database elements may deliver a reminder instruction for smart package content use, for example in the event that the database elements have not received information from the smart package regarding the smart package content use within a predetermined time frame. It should be noted that the reminder instruction can be delivered either to the smart package or to the user's mobile communicator or to both. The instruction can also be delivered to some other terminal unit, such as for example to a guardian of the medication package user or to a party looking after his/her health, or to an insurance company.

According to one embodiment, the smart package may comprise communication elements for receiving information from the database elements, such as for example for receiving a reminder instruction. The smart package may additionally comprise an attention getter, such as a led component or a sound producing element, by way of which it can produce a reminder to the user for smart package content use on the basis of instruction information received thereby from the database elements.

In particular, an alarm may be triggered by a situation in which a pill was taken from the smart package at a certain location, but the user or his/her mobile communicator was located at this particular moment at least a predetermined distance away from said location of the smart package. The alarm can be for example a notice or request type alarm to the user and/or to some third party, such as caretaking staff, a guardian, or an insurance company.

The method of the invention comprises providing database elements, wherein the database elements comprise means for linking the smart package identification information to the smart package user in the database elements, as well as elements for linking a smart package content use instruction to said smart package identification information in the database elements, as well as communication elements for delivering content use relating instructions either to the smart package and/or to the user's other terminal unit. Furthermore, the method comprises providing the smart package comprising recyclable material with a data transfer module functional in a mobile communication network and with a docking unit of recyclable material for docking said data transfer module to the package in releasable and electrical manner for the duration of package use. Still further, the method comprises storing identification information in the database elements regarding the system's smart package, such as for example information about a SIM card included in the smart package and/or other package identifying information. In addition, in the database elements can be stored information for using said smart package content, such as for example a medical prescription along with its dosage instructions and time attributes, according to which time intervals the content should be used as per instruction. The information regarding a smart package content use can be linked with said smart package identification information in the database elements. The method also comprises linking the smart package identification information to the smart package user in the database elements, and linking the smart package content use instruction to said smart package identification information in the database elements. By way of communication elements, it is possible to deliver information from said smart package to said database elements, regarding for example the identification of a smart package or a communication module included therein, as well as the removal of a pill, whereby said smart package can be identified by the database elements on the basis of information it has supplied by way of said communication elements, such as on the basis of the ID information of a package or the information of a SIM card included in the module, such as a GSM module, present in the package. The method further comprises comparing pieces of location data regarding both the smart package and the smart package user's other terminal unit, and producing an alarm in case said pieces of location data deviate from each other more than what has been prescribed, at a time when, according to the smart package content use instruction, some content of the smart package should have been removed from said smart package.

The backing or the smart package of the invention comprises preferably also a power source for supplying electric power to said components of the package. In addition, it comprises a data processing medium, such as a microcircuit, for measuring electrical changes in the backing. Said data processing medium can be adapted to identify also said removed object on the basis of a specific electrical change brought about by said removed object in one of the electrical lines or circuits of said backing or smart package.

It should be noted that the smart package used in the monitoring system, or at least its backing which has pill identifying elements and components, is most preferably a fiber- or plastic-based backing, comprising cardboard or paper or the like fiber-based material. It should particularly be noted that, according to one preferred embodiment, the backing comprises a docking unit for docking said data transfer elements, such as a GSM module or the like, to the backing for example for the duration of medication package use, whereby, after the medication package use, the data transfer elements can be disengaged from the package for connecting the same for example to a new package, and the spent package can be recycled for example along with recyclable board, or incinerated. The docking unit can be fabricated from the same recyclable and environmentally sound material as the smart package or its backing as well, and its conductor lines can be constructed by methods of printing technology similar to those used for printing its other components as well.

### DESRIPTION OF FIGURES

Preferred embodiments of the invention will be described in the following segment slightly more precisely with reference to the accompanying figures, in which
- fig. 1: shows, according to one preferred embodiment of the invention, one exemplary monitoring arrangement, and
- fig. 2: shows, according to one preferred embodiment of the invention, one smart package of the monitoring arrangement.

### DETAILED DESCRIPTION OF FIGURES

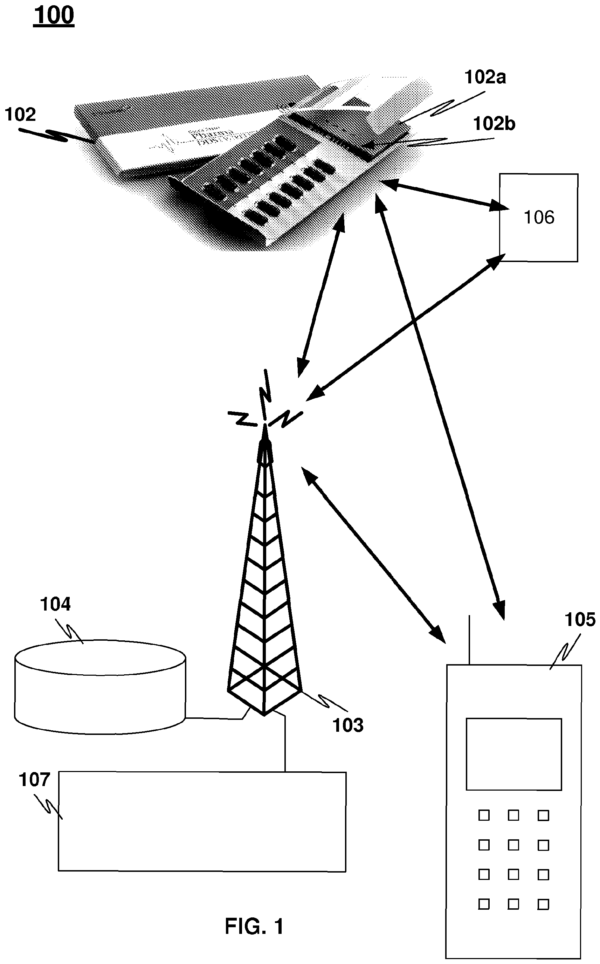

Fig. 1 shows one exemplary monitoring system 100 according to one preferred embodiment of the invention for monitoring use of the contents, such as medical pills, of a smart package 102, wherein the monitoring system 100 comprises not only a smart package but also database elements 104. The monitoring system may also comprise the smart package user's other terminal unit, such as a mobile communicator 105.

The smart package 102 comprises removable content units, such a pill type or tablet type objects, for example medical pills, as well as most preferably wireless communication elements 102a, which are most preferably implemented by means of a module. The module can be for example a GSM, GPRS or UMTS module capable of communicating with a base station included in a mobile communication system. Alternatively or additionally, the package may also feature a data transfer solution based on short range RF technology, such as RFFID or NFC technique, by way of which the package is able to communicate with some other RFIS, NFC or the like device 106. The package may also include a wired data transfer medium, such as a USB interface. According to one embodiment, the device 106 may be for example a device measuring the package user's physiological functions, from which the smart package can inquire measurement data for example in conjunction with the removal of a content unit and supply the database elements not only with information regarding the removal of a content unit but also information regarding the user's functions, such as for example heart rate information or other information, which it receives after inquiring from the device 106. It may also be the case that the sensors measuring physiological functions converse with the device 106 in a centralized manner, and the device 106 then delivers the information, which is has collected from the sensors, in an assembled form to the smart package as the latter inquires information.

It should particularly be noted that, according to one preferred embodiment, the backing comprises a docking unit 102b for docking said data transfer elements to the backing for example for the duration of medication package use, whereby, after the medication package use, the data transfer elements can be disengaged from the package for connecting the same for example to a new package, and the spent package can be recycled for example along with recyclable cardboard, or incinerated.

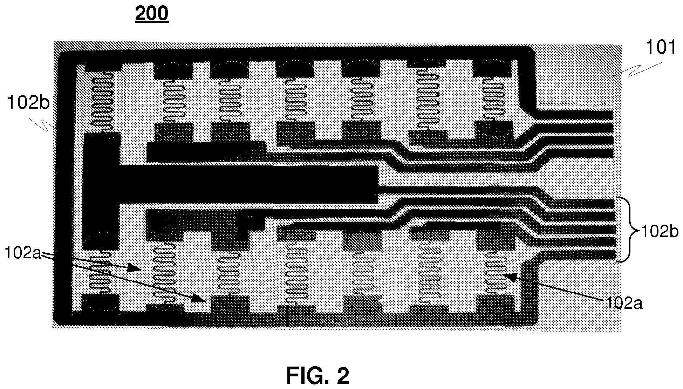

The smart package (as shown in fig. 2) is preferably adapted to detect said removal of a pill and to supply information to said database elements 104 by way of said communication elements 102a, 103, regarding a removal of said content unit. Said smart package can be identified by means of said database elements 104 on the basis of information it has delivered by way of said communication elements. The smart package may transmit for example a package identifying character string or, alternatively, its communication elements 102a may transmit specific identification data, such as the data of a SIM card, in connection with transmitting other information.

Fig. 2 shows one backing 200 for the smart package 102 of the monitoring system 100 according to one preferred embodiment of the invention, wherein the package is adapted to record a removal of pills by means of detection elements 102, 103 provided in the backing 200. The detection elements comprise electrical components 102a, 102b, which are most preferably pressed onto the backing 200 with methods of printing technology. The removal of a pill from the backing is adapted to bring about a structural change in at least one printed component, such that the change produces an electrically measurable change in at least one of said electrical components printed on the backing. The electrical printed component can be for example a resistor or a capacitor and said electrical change can be for example a change of resistance or capacitance. A component of the backing can be coupled to be a part of some electric circuit of the backing, such as an oscillator, whereby the change of resistance or capacitance brings about a change in the oscillator's frequency, the magnitude of which is proportional to the magnitude of the change in resistance or capacitance. Respectively, since every pill has a designated component, whose electrical property, such as resistance or capacitance, deviates from other components of the backing, the change-producing component, and thereby the pill corresponding to that component, can be identified on the basis of the magnitude of a change in resistance or capacitance or a change in the oscillator's frequency.

Described above are just a few embodiments for a solution of the invention. The principle according to the invention can naturally be varied within the scope of protection defined by the claims, for example in terms of implementation details as well as fields of use. The smart package of the invention is most preferably a fiber- or plastic based package. In some application examples, the fiber- or plastic based package material comprises cardboard. Alternatively, the fiber- or plastic-based package material may also comprise for example paper or the like. As an option to fiber, one embodiment of the invention enables using also plastics, polymer films, or the like. In reference to exemplary embodiments of the invention, it should indeed be noted that fiber or plastic may refer at least to the former and equivalents thereof. Hence, a functional backing can obtained by using almost any insulating and flat blank, fiber, fibrous mesh, textile, wallpaper, coating, biomaterial, ceramic coaster, glass, aluminum foil, etc. The backing 100 may be coated or uncoated, as well as wood-based or not wood-based. Thus, every substrate has its inherent benefits and drawbacks, which apply and are relevant to the required package. In terms of its temperature, the substrate can be for example such that the melting and reshaping point withstands temperatures of 200°C, temperatures particularly less than 250°C or preferably less than 300°C. According to one embodiment, the smart package or the backing can be a blister package in itself, such as for example an aluminum foil coated with plastic or the like, upon which the components are pressed or printed for example by inkjet technique.

It should further be noted that the smart package used in the monitoring system of the invention can be for example a medication package, but also some other similar type package making use of a backing described in this document. This type of other smart packages can be for example racks and packages and shipping pallets intended for shipping and/or storing single-unit articles, such as yogurt containers or other one-by-one removable content units, whereby embodiments of the invention enable also carrying out inventory bookkeeping. The smart package of the invention, which is used for storage, for example a storage pallet or the like, may comprise a plurality of singly packed content units and may notify for example the accountant by way of the communication elements about a removal of content units, the number of such units, and that said smart package, i.e. in this case the storage pallet, is running out of such units.

According to one embodiment, the smart package or its backing can be for example a fiber-based layer, which is for example a bottom layer or part of it for a medication package or the like. According to one embodiment, the smart package is a blister package, wherein every blister comprises for example one medicine or dose of medicine, and wherein the blisters are arranged relative to the backing in such a way that each blister is matched by at least one printed electrical component set forth in this document. The removal of medicine is most preferably performed by pressing on the blister, whereby the medicine forces itself through the backing layer and at the same changes the structure of a corresponding component, such as for example breaks the component's electric circuit. According to another example, the smart package or its backing can be for example a backing of cardboard or made of some other appropriate material and intended for shipping and/or storing content units.

## Claims

1. A monitoring system (100) for monitoring smart package content use, wherein the monitoring system comprises:
- a smart package (102) of recyclable material, configured to comprise removable content units as well comprising a data transfer module (102a) functional in a mobile communication network and a docking unit of recyclable material for docking said data transfer module to the package in releasable and electrical manner for the duration of package use, and
- database elements (104),
wherein the smart package is adapted to detect said removal of a content unit and to supply information to said database elements by way of said communication elements, regarding a removal of said content unit, whereby said smart package is capable of being identified by means of the database elements on the basis of information it has supplied by way of said communication elements,.
further wherein the database elements comprise means for linking the smart package identification information to the smart package user in the database elements, as well as elements for linking a smart package content use instruction to said smart package identification information in the database elements, as well as communication elements for delivering content use relating instructions either to the smart package and/or to the user's other terminal unit, in which case the monitoring system comprises also said terminal unit,
and finally wherein the monitoring system is adapted to compare location data of both the smart package and the user's other terminal unit and to produce an alarm in the event that said pieces of location data deviate from each other more than what has been prescribed, at a time when, according to the smart package content use instruction, some contents of the smart package should have been removed from said smart package.

2. A monitoring system according to claim 1, wherein the smart package is provided for each content unit, such a pill type or tablet type object, with a specific sensing element for detecting a removal of this particular content unit, the smart package being adapted to detect and identify, by means of said sensing elements, the removal of a specific content unit corresponding to the sensing device, and to supply information by means of communication elements, regarding the removal of just this particular content unit.

3. A monitoring system according to either of the preceding claims, wherein the sensing element provided for a content unit comprises a component, such as a resistor, capacitor, and/or switch, which is fabricated by methods of printing technology, and wherein the specific electrical values, such as resistance or capacitance, of at least two printed components present in the backing are adapted by methods of printing technology to be different from each other, such that the removal of a content unit brings about a change in the structure of a specific component, whereby the removable content unit producing said change is adapted to be identified on the basis of a change in the specific electrical value inherent to said component.

4. A monitoring system according to any of the preceding claims, wherein the communication elements comprise wireless communication elements, such as a GSM, GPRS or UMTS module or some other data transfer module functional in a mobile communication network, and wherein the smart package additionally comprises smart package identifying elements, such as for example a SIM card.

5. A monitoring system according to any of the preceding claims, wherein the smart package comprises positioning elements, such as for example a module receiving location data delivered by a GPS receiver or most preferably by the cellular information of a mobile communication network, such as for example a module as set forth in claim 4.

6. A monitoring system according to any preceding claim, wherein the database elements are adapted to deliver a reminder instruction for smart package content use in case of not having received information from the smart package regarding the smart package content use within a predetermined time frame.

7. A monitoring system according to any preceding claim, wherein the smart package comprises communication elements for receiving information from the database elements, as well as an attention getter, such as a led component or a sound producing element, for producing a reminder to the user for smart package content use on the basis of instruction information received thereby from the database elements.

8. A method for monitoring smart package content use, wherein the smart package comprises removable content units, such as pill type or tablet type objects, wherein the method comprises
- providing database elements, wherein the database elements comprise means for linking the smart package identification information to the smart package user in the database elements, as well as elements for linking a smart package content use instruction to said smart package identification information in the database elements, as well as communication elements for delivering content use relating instructions either to the smart package and/or to the user's other terminal unit,
- providing the smart package comprising recyclable material with a data transfer module functional in a mobile communication network and with a docking unit of recyclable material for docking said data transfer module to the package in releasable and electrical manner for the duration of package use,
- storing identification information in the database elements, regarding said smart package, such as for example information about a SIM card included in the smart package,
- storing information in the database elements for using said smart package content, such as for example a medical prescription along with its dosage instructions and time attributes, according to which time intervals the content should be used as per instruction,
- linking said information regarding the smart package content use with said smart package identification information in the database elements,
- linking the smart package identification information to the smart package user in the database elements,
- linking the smart package content use instruction to said smart package identification information in the database elements,
- delivering information by way of the communication elements from said smart package to said database elements, regarding the removal of a content unit, said smart package being identifiable by the database elements on the basis of information it has supplied delivered by way of said communication elements, and
- comparing pieces of location data regarding both the smart package and the smart package user's other terminal unit, and producing an alarm in case said pieces of location data deviate from each other more than what has been prescribed, at a time when, according to the smart package content use instruction, some content of the smart package should have been removed from said smart package.

9. A method according to claim 8, which comprises monitoring every removal of the smart package's content unit, such as a pill type or tablet type object, by means of a designated, identifiable sensing element, the smart package detecting and identifying, by means of said sensing elements, the removal of a specific content unit corresponding to a sensing element, and delivering information by way of communication elements, regarding the removal of just this particular content unit.

10. A method according to either of claims 8-9, which comprises delivering instructions regarding content use from the database elements either to the smart package and/or to the user's other terminal unit, such as for example his/her mobile communicator.

11. A method according to any of claims 8-10, which comprises delivering a reminder instruction from the database elements to the smart package and/or to the user's other terminal unit, such as for example his/her mobile communicator, for using the smart package content in the event that the database elements have not received from the smart package information regarding smart package content use within a predetermined time frame.

12. A method according to any of claims 8-11, wherein the smart package receives information from the database elements by way of communication elements and produces, by means of an attention getter, such as a led component or a sound producing element, a reminder to the user for using the smart package content on the basis of instruction information it has received from the database elements.

## Patentansprüche

1. Überwachungssystem (100) zum Überwachen der Verwendung von Inhalten intelligenter Pakete, wobei das Überwachungssystem Folgendes umfasst:
- ein intelligentes Paket (102) aus wiederverwertbarem Material, das konfiguriert ist, um entfernbare Inhaltseinheiten sowie ein Datenübertragungsmodul (102a) zu umfassen, das in einem Mobilkommunikationsnetzwerk funktionsfähig ist, und eine Andockeinheit aus wiederverwertbarem Material zum Andocken des Datenübertragungsmoduls an das Paket in lösbare und elektrische Art und Weise für die Dauer der Paketverwendung, und
- Datenbankelemente (104),
wobei das intelligente Paket angepasst ist, um das Entfernen einer Inhaltseinheit zu erfassen und den Datenbankelementen Informationen über die Kommunikationselemente bezüglich eines Entfernens der Inhaltseinheit bereitzustellen, wobei das intelligente Paket mittels der Datenbankelemente auf der Grundlage von Informationen identifiziert werden kann, die es über die Kommunikationselemente geliefert hat,
ferner, wobei die Datenbankelemente Folgendes umfasst: Mittel zum Verknüpfen der Identifikationsinformationen intelligenter Pakete mit dem Benutzer intelligenter Pakete in den Datenbankelementen, sowie Elemente zum Verknüpfen einer Inhaltsverwendungsanweisung intelligenter Pakete mit den Identifikationsinformationen intelligenter Pakete in den Datenbankelementen, sowie Kommunikationselemente zur Übermittlung von Anweisungen zur Verwendung von Inhalten entweder an das intelligente Paket und / oder an die andere Endeinheit des Benutzers, in welchem Fall das Überwachungssystem auch die Endeinheit umfasst,
und schließlich, wobei das Überwachungssystem angepasst ist, um Standortdaten sowohl des intelligenten Pakets als auch der anderen Endeinheit des Benutzers zu vergleichen und einen Alarm für den Fall auszulösen, dass die Standortdaten stärker voneinander abweichen als vorgeschrieben, zu einem Zeitpunkt, zu dem gemäß der Anweisung zur Verwendung von Inhalten intelligenter Pakete einige Inhalte des intelligenten Pakets entfernt werden sollten.

2. Überwachungssystem nach Anspruch 1, wobei das intelligente Paket für jede Inhaltseinheit, wie ein Objekt vom Pillentyp oder Tablettentyp, mit einem spezifischen Erfassungselement zum Erfassen einer Entfernung dieser bestimmten Inhaltseinheit bereitgestellt wird, wobei das intelligente Paket angepasst ist, um mittels der Erfassungselemente das Entfernen einer spezifischen Inhaltseinheit zu erfassen und zu identifizieren, die der Erfassungsvorrichtung entspricht und Informationen mittels Kommunikationselementen bezüglich der Entfernung nur dieser bestimmten Inhaltseinheit bereitzustellen.

3. Überwachungssystem nach einem der vorhergehenden Ansprüche, wobei das für eine Inhaltseinheit vorgesehene Erfassungselement eine Komponente wie einen Widerstand, einen Kondensator und / oder einen Schalter umfasst, die durch Verfahren der Drucktechnologie hergestellt wird, und wobei die spezifischen elektrischen Werte, wie Widerstand oder Kapazität, von mindestens zwei gedruckten Komponenten, die in dem Träger vorhanden sind, durch Verfahren der Drucktechnologie angepasst werden, um sich voneinander zu unterscheiden, so dass das Entfernen einer Inhaltseinheit eine Änderung in der Struktur einer bestimmten Komponente bewirkt, wobei die entfernbare Inhaltseinheit, die die Änderung erzeugt, angepasst ist, um auf der Basis einer Änderung des spezifischen elektrischen Wertes, der der Komponente inhärent ist, identifiziert zu werden.

4. Überwachungssystem nach einem der vorhergehenden Ansprüche, wobei die Kommunikationselemente drahtlose Kommunikationselemente umfassen, wie beispielsweise ein GSM-, GPRS- oder UMTS-Modul oder ein anderes Datenübertragungsmodul, das in einem Mobilkommunikationsnetzwerk funktionsfähig ist, und wobei das Smart-Paket zusätzlich Smart-Paket-Identifizierungselemente, wie beispielsweise eine SIM-Karte umfasst.

5. Überwachungssystem nach einem der vorhergehenden Ansprüche, wobei das intelligente Paket Positionierungselemente umfasst, wie beispielsweise ein Modul, das Standortdaten empfängt, die von einem GPS-Empfänger oder am bevorzugtesten von der zellularen Information eines Mobilkommunikationsnetzwerks geliefert werden, wie zum Beispiel ein Modul nach Anspruch 4.

6. Überwachungssystem nach einem vorhergehenden Anspruch, wobei die Datenbankelemente angepasst sind, um eine Erinnerungsanweisung für die Verwendung von Inhalten intelligenter Pakete zu liefern, falls keine Informationen von dem intelligenten Paket bezüglich der Verwendung von Inhalten intelligenter Pakete innerhalb eines vorbestimmten Zeitrahmens empfangen wurden.

7. Überwachungssystem nach einem vorhergehenden Anspruch, wobei das intelligente Paket Kommunikationselemente zum Empfangen von Informationen von den Datenbankelementen sowie einen Aufmerksamkeitsgewinner, wie eine LED-Komponente oder ein Tonerzeugungselement zum Erzeugen einer Erinnerung an den Benutzer für die Verwendung von Inhalten intelligenter Pakete auf der Basis von Anweisungsinformationen umfasst, die dadurch von den Datenbankelementen empfangen werden.

8. Verfahren zum Überwachen der Verwendung von Inhalten intelligenter Pakete, wobei das intelligente Paket entfernbare Inhaltseinheiten umfasst, wie beispielsweise Objekte vom Pillentyp oder Tablet-Typ, wobei das Verfahren Folgendes umfasst
- Bereitstellen von Datenbankelementen, wobei die Datenbankelemente Mittel zum Verknüpfen der Identifikationsinformationen intelligenter Pakete mit dem Benutzer intelligenter Pakete in den Datenbankelementen sowie Elemente zum Verknüpfen einer Inhaltsverwendungsanweisung intelligenter Pakete mit den Identifikationsinformationen intelligenter Pakete in den Datenbankelementen sowie Kommunikationselemente zur Übermittlung von Anweisungen zur Verwendung von Inhalten entweder an das intelligente Paket und / oder an die andere Endeinheit des Benutzers umfassen;
- Bereitstellen des intelligenten Pakets, das wiederverwertbares Material umfasst, mit einem Datenübertragungsmodul, das in einem Mobilkommunikationsnetzwerk funktionsfähig ist, und mit einer Andockeinheit aus wiederverwertbarem Material zum Andocken des Datenübertragungsmoduls an das Paket auf lösbare und elektrische Weise für die Dauer der Paketverwendung,
- Speichern von Identifikationsinformationen in den Datenbankelementen in Bezug auf das intelligente Paket, wie beispielsweise Informationen über eine im intelligenten Paket enthaltene SIM-Karte,
- Speichern von Informationen in den Datenbankelementen zur Verwendung des Inhalts des intelligenten Pakets, wie beispielsweise einer ärztlichen Verschreibung zusammen mit ihren Dosierungsanweisungen und Zeitattributen, wobei nach deren Zeitintervallen der Inhalt gemäß Anweisung verwendet werden soll,
- Verknüpfen der Informationen bezüglich der Verwendung von Inhalten intelligenter Pakete mit den Identifikationsinformationen intelligenter Pakete in den Datenbankelementen,
- Verknüpfen der Informationen zur Identifizierung intelligenter Pakete mit dem Benutzer intelligenter Pakete in den Datenbankelementen,
- Verknüpfen der Anweisung zur Verwendung des Inhalts des intelligenten Pakets mit den Informationen zur Identifizierung des intelligenten Pakets in den Datenbankelementen;
- Übermitteln von Informationen über die Kommunikationselemente aus dem intelligenten Paket an die Datenbankelemente bezüglich der Entfernung einer Inhaltseinheit, wobei das intelligente Paket durch die Datenbankelemente auf der Grundlage von Informationen identifizierbar ist, die es über die Kommunikationselemente geliefert hat, und
- Vergleichen von Standortdaten sowohl für das intelligente Paket als auch für die andere Endeinheit des Benutzers des intelligenten Pakets, und Erzeugen eines Alarms für den Fall, dass die Standortdaten stärker voneinander abweichen als vorgeschrieben, zu einem Zeitpunkt, zu dem gemäß der Anweisung zur Verwendung des Inhalts des intelligenten Pakets ein Teil des Inhalts des intelligenten Pakets aus dem intelligenten Paket entfernt werden sollte.

9. Verfahren nach Anspruch 8, umfassend das Überwachen jeder Entfernung der Inhaltseinheit des intelligenten Pakets, wie beispielsweise eines Pillen- oder Tablettenobjekts, mittels eines bezeichneten identifizierbaren Erfassungselements, wobei das intelligente Paket, das mittels der Erfassungselemente das Entfernen einer bestimmten Inhaltseinheit, die einem Erfassungselement entspricht, erfasst und identifiziert, und Bereitstellen von Informationen über Kommunikationselemente bezüglich der Entfernung nur dieser bestimmten Inhaltseinheit.

10. Verfahren nach einem der Ansprüche 8 bis 9, das das Liefern von Anweisungen bezüglich der Verwendung von Inhalten von den Datenbankelementen entweder an das intelligente Paket und / oder an die andere Endeinheit des Benutzers umfasst, wie beispielsweise seinen / ihren mobilen Kommunikator.

11. Verfahren nach einem der Ansprüche 8 bis 10, umfassend das Liefern einer Erinnerungsanweisung von den Datenbankelementen an das intelligente Paket und / oder an die andere Endeinheit des Benutzers, wie beispielsweise seinen / ihren mobilen Kommunikator zur Verwendung des Inhalts des intelligenten Pakets für den Fall, dass die Datenbankelemente innerhalb eines vorgegebenen Zeitrahmens keine Informationen über die Verwendung des Inhalts des intelligenten Pakets von dem intelligenten Paket erhalten haben.

12. Verfahren nach einem der Ansprüche 8 bis 11, wobei das intelligente Paket Informationen von den Datenbankelementen über Kommunikationselemente empfängt und mittels eines Aufmerksamkeitsempfängers, wie beispielsweise einer LED-Komponente oder eines Ton erzeugenden Elements, eine Erinnerung an den Benutzer für die Verwendung des Inhalts des intelligenten Pakets auf der Grundlage von Anweisungsinformationen erzeugt, die es von den Datenbankelementen erhalten hat.

## Revendications

1. Système de surveillance (100) permettant de surveiller l'utilisation du contenu d'un emballage intelligent, dans lequel le système de surveillance comprend :
- un emballage intelligent (102) en matériau recyclable, configuré pour comprendre des unités de contenu amovibles comprenant également un module de transfert de données (102a) fonctionnel dans un réseau de communication mobile et une unité d'accueil en matériau recyclable pour accueillir ledit module de transfert de données sur l'emballage de manière libérable et électrique pendant la durée d'utilisation de l'emballage, et
- des éléments de base de données (104),
dans lequel l'emballage intelligent est conçu pour détecter ledit retrait d'une unité de contenu et pour fournir des informations auxdits éléments de base de données au moyen desdits éléments de communication, concernant un retrait de ladite unité de contenu, moyennant quoi ledit emballage intelligent est capable d'être identifié au moyen des éléments de base de données sur la base d'informations qu'il a fournies au moyen desdits éléments de communication,
en outre dans lequel les éléments de base de données comprennent des moyens pour lier les informations d'identification d'emballage intelligent à l'utilisateur d'emballage intelligent dans les éléments de base de données, ainsi que des éléments pour lier une instruction d'utilisation du contenu d'un emballage intelligent auxdites informations d'identification d'emballage intelligent dans les éléments de base de données, ainsi que des éléments de communication pour délivrer des instructions relatives à l'utilisation du contenu soit au module intelligent et/ou à l'autre unité terminale de l'utilisateur, auquel cas le système de surveillance comprend également ladite unité terminale,
et enfin dans lequel le système de surveillance est conçu pour comparer des données de localisation à la fois de l'emballage intelligent et de l'autre unité terminale de l'utilisateur et pour produire une alarme dans le cas où lesdites parties de données de localisation diffèrent les unes des autres plus que ce qui a été prescrit, à un moment où, selon l'instruction d'utilisation du contenu d'un emballage intelligent, certains contenus de l'emballage intelligent devraient avoir été retirés dudit emballage intelligent.

2. Système de surveillance selon la revendication 1, dans lequel l'emballage intelligent est fourni pour chaque unité de contenu, comme un objet de type pilule ou de type comprimé, avec un élément de détection spécifique pour détecter un retrait de cette unité de contenu particulière, l'emballage intelligent étant conçu pour détecter et identifier, au moyen desdits éléments de détection, le retrait d'une unité de contenu spécifique correspondant au dispositif de détection, et pour fournir des informations au moyen d'éléments de communication, concernant le retrait de cette seule unité de contenu particulière.

3. Système de surveillance selon l'une ou l'autre des revendications précédentes, dans lequel l'élément de détection fourni pour une unité de contenu comprend un composant, comme une résistance, un condensateur et/ou un commutateur, qui est fabriqué par des procédés de technologie d'impression, et dans lequel les valeurs électriques spécifiques, comme la résistance ou la capacité, d'au moins deux composants imprimés présents dans le support sont conçues par des procédés de technologie d'impression pour être différentes les unes des autres, de sorte que le retrait d'une unité de contenu entraîne un changement dans la structure d'un composant spécifique, moyennant quoi l'unité de contenu amovible produisant ledit changement est conçue pour être identifiée sur la base d'un changement de la valeur électrique spécifique inhérente audit composant.

4. Système de surveillance selon l'une quelconque des revendications précédentes, dans lequel les éléments de communication comprennent des éléments de communication sans fil, comme un module GSM, GPRS ou UMTS ou un autre module de transfert de données fonctionnel dans un réseau de communication mobile, et dans lequel l'emballage intelligent comprend en outre des éléments identifiant un emballage intelligent, comme par exemple une carte SIM.

5. Système de surveillance selon l'une quelconque des revendications précédentes, dans lequel l'emballage intelligent comprend des éléments de positionnement, comme par exemple un module recevant des données de localisation délivrées par un récepteur GPS ou de manière préférée entre toutes par les informations cellulaires d'un réseau de communication mobile, comme par exemple un module selon la revendication 4.

6. Système de surveillance selon une quelconque revendication précédente, dans lequel les éléments de base de données sont conçus pour délivrer une instruction de rappel pour une utilisation du contenu d'un emballage intelligent dans le cas où des informations n'ont pas été reçues depuis l'emballage intelligent concernant l'utilisation du contenu d'un emballage intelligent dans une période de temps prédéterminée.

7. Système de surveillance selon une quelconque revendication précédente, dans lequel l'emballage intelligent comprend des éléments de communication pour recevoir des informations depuis les éléments de base de données, ainsi qu'un capteur d'attention, comme un composant DEL ou un élément de production de son, pour produire un rappel à l'utilisateur pour une utilisation du contenu d'un emballage intelligent sur la base d'informations d'instruction ainsi reçues depuis les éléments de base de données.

8. Procédé permettant de surveiller l'utilisation du contenu d'un emballage intelligent, dans lequel l'emballage intelligent comprend des unités de contenu amovibles, comme des objets de type pilule ou de type comprimé, dans lequel le procédé comprend
- la fourniture d'éléments de base de données, dans lequel les éléments de base de données comprennent des moyens pour lier les informations d'identification d'emballage intelligent à l'utilisateur d'emballage intelligent dans les éléments de base de données, ainsi que des éléments pour lier une instruction d'utilisation du contenu d'un emballage intelligent auxdites informations d'identification d'emballage intelligent dans les éléments de base de données, ainsi que des éléments de communication pour délivrer des instructions relatives à l'utilisation du contenu soit au module intelligent et/ou à l'autre unité terminale de l'utilisateur,
- la fourniture de l'emballage intelligent comprenant un matériau recyclable avec un module de transfert de données fonctionnel dans un réseau de communication mobile et avec une unité d'accueil en matériau recyclable pour accueillir ledit module de transfert de données sur l'emballage de manière libérable et électrique pendant la durée d'utilisation de l'emballage,
- le stockage d'informations d'identification dans les éléments de la base de données, concernant ledit emballage intelligent, comme par exemple des informations sur une carte SIM incluse dans l'emballage intelligent,
- le stockage d'informations dans les éléments de base de données pour utiliser ledit contenu d'un emballage intelligent, comme par exemple une prescription médicale avec ses instructions de dosage et ses attributs de temps, selon les intervalles de temps auxquels le contenu doit être utilisé conformément à l'instruction,
- la liaison desdites informations concernant l'utilisation du contenu d'un emballage intelligent avec lesdites informations d'identification d'emballage intelligent dans les éléments de la base de données,
- la liaison des informations d'identification d'emballage intelligent à l'utilisateur de l'emballage intelligent dans les éléments de base de données,
- la liaison de l'instruction d'utilisation du contenu d'un emballage intelligent auxdites informations d'identification d'emballage intelligent dans les éléments de base de données,
- la délivrance d'informations au moyen des éléments de communication depuis ledit emballage intelligent auxdits éléments de base de données, concernant le retrait d'une unité de contenu, ledit emballage intelligent étant identifiable par les éléments de base de données sur la base d'informations qu'il a fournies délivrées au moyen desdits éléments de communication, et
- la comparaison de parties de données de localisation concernant à la fois l'emballage intelligent et l'autre unité terminale de l'utilisateur, et la production d'une alarme dans le cas où lesdites parties de données de localisation diffèrent les unes des autres plus que ce qui a été prescrit, à un moment où, selon l'instruction d'utilisation du contenu d'un emballage intelligent, certains contenus de l'emballage intelligent devraient avoir été retirés dudit emballage intelligent.

9. Procédé selon la revendication 8, qui comprend la surveillance de chaque retrait de l'unité de contenu de l'emballage intelligent, comme un objet de type pilule ou de type comprimé, au moyen d'un élément de détection désigné et identifiable, l'emballage intelligent détectant et identifiant, au moyen desdits éléments de détection, le retrait d'une unité de contenu spécifique correspondant à un élément de détection, et la délivrance d'informations au moyen d'éléments de communication, concernant le retrait de cette seule unité de contenu particulière.

10. Procédé selon l'une ou l'autre des revendications 8 et 9, qui comprend la délivrance d'instructions concernant l'utilisation du contenu depuis les éléments de base de données soit à l'emballage intelligent et/ou à l'autre unité terminale de l'utilisateur, comme par exemple son communicateur mobile.

11. Procédé selon l'une quelconque des revendications 8 à 10, qui comprend la délivrance d'une instruction de rappel depuis les éléments de base de données à l'emballage intelligent et/ou à l'autre unité terminale de l'utilisateur, comme par exemple son communicateur mobile, pour utiliser le contenu d'un emballage intelligent dans le cas où les éléments de base de données n'ont pas reçu depuis l'emballage intelligent des informations concernant l'utilisation du contenu d'un emballage intelligent dans une période de temps prédéterminée.

12. Procédé selon l'une quelconque des revendications 8 à 11, dans lequel l'emballage intelligent reçoit des informations depuis les éléments de base de données au moyen d'éléments de communication et produit, au moyen d'un capteur d'attention, comme un composant DEL ou un élément de production de son, un rappel à l'utilisateur pour utiliser le contenu d'un emballage intelligent sur la base d'informations d'instruction qu'il a reçues depuis les éléments de base de données.
